(19) **Europäisches Patentamt European Patent Office Office européen des brevets**

(11) **EP 3 740 166 B1**

(12) **EUROPEAN PATENT SPECIFICATION**

(45) Date of publication and mention of the grant of the patent:
**05.01.2022 Bulletin 2022/01**

(51) Int Cl.:
*A61F 2/46* (2006.01)    *A61B 17/92* (2006.01)
*A61B 90/00* (2016.01)

(21) Application number: **19700154.8**

(86) International application number:
**PCT/EP2019/050314**

(22) Date of filing: **08.01.2019**

(87) International publication number:
**WO 2019/141537 (25.07.2019 Gazette 2019/30)**

(54) **SURGICAL IMPACTION APPARATUS**

CHIRURGISCHE IMPAKTIONSVORRICHTUNG

APPAREIL CHIRURGICAL D'IMPACTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.01.2018 GB 201800701**

(43) Date of publication of application:
**25.11.2020 Bulletin 2020/48**

(73) Proprietor: **DePuy Ireland Unlimited Company County Cork (IE)**

(72) Inventors:
• **ADEKANMBI, Isaiah**
  **Leeds, West Yorkshire LS11 8DT (GB)**
• **EHTESHAMI, Zahra**
  **Leeds, West Yorkshire LS11 8DT (GB)**
• **HUNT, Christopher**
  **Leeds, West Yorkshire LS11 8DT (GB)**

(74) Representative: **Burton, Nick**
  **Murgitroyd & Company Leeds**
  **Arena Point**
  **Merrion Way**
  **Leeds LS2 8PA (GB)**

(56) References cited:
**EP-A1- 3 260 088      US-A- 5 836 891**
**US-A1- 2015 142 372      US-A1- 2015 282 856**

## Description

FIELD OF THE INVENTION

This invention relates to a surgical impaction apparatus.

BACKGROUND OF THE INVENTION

In the field of Orthopaedics, instrumented hammers designed for test engineers and clinicians can enable mechanical and structural analysis as well as trouble shooting of manual hammer strikes during insertion of a component into an opening in a bone by impaction.

Currently available systems typically use a hammer equipped with a force sensor to measure the forces applied to an insertion instrument or test object by the hammer. This can help to provide a measure of the object's structural-mechanical response due to the hammer strikes.

US 2008/0125671 describes a method and system for monitoring impaction of a femoral component of a hip prosthesis into a femur in which impaction data generated during the impaction of the femoral component into the femur is received from at least one measurement transducer attached to the femoral component and is normalized by a data acquisition and analysis device. An impaction monitoring metric is calculated based on the normalized impaction data, and femoral component fit and stability data is then generated and output to a user interface based on the impaction monitoring metric.

US 2015/0282856 and EP 2 923 677 describe a device assisting with the placement of an orthopaedic instrument intended to be inserted in a receiving bone by impaction, by means of an impactor comprising at least one striking surface designed to come into contact with an impact surface coupled to the orthopaedic instrument in order to apply an impaction force to the orthopaedic instrument, the device being characterized in that it comprises a force sensor able to measure, upon each impact, the impaction force applied by the impactor to the impact surface and to supply a measurement signal indicative of the temporal variation of the applied impaction force, the force sensor being connected to processing means designed to determine, on the basis of the temporal variation of the impaction force supplied for each impact in succession, an indicator representative of the degree of insertion of the orthopaedic instrument in the receiving bone.

An Article by Michel, A., Bosc, R., Sailhan , F., Vayron, R., Haiat, G., entitled "Ex vivo estimation of cementless acetabular cup stability using an impact hammer", published in Medical Engineering and Physics 38 (2016) 80-86, investigates whether acetabular cup (AC) implant primary stability can be evaluated using the signals obtained with an impact hammer. A hammer equipped with a force sensor was used to impact the AC implant in 20 bovine bone samples.

WO 2018/085423 A1 describes systems and methods to display an indication of an impact force from a driving device. A method is described that includes displaying the indication of the impact force with a light. The light may be located on the driving device, a component attached to the driving device, a user interface, or the like. Different colors may be used to indicate different impact forces. For example, a first light color may correspond to an impact force below a first threshold, a second light color may correspond to an impact force above a second threshold, and a third light color may correspond to an impact force between the thresholds. The impact force may be detected using a sensor, which may output a voltage to cause the illumination.

EP 3 260 088 A1 describes a system that comprises: - at least one vibrational sensor which is operable to produce data each time an impact application device applies an impact to the prosthetic component during assembly between the prosthetic component and with the support member, the produced data representing acoustic vibrations generated in the air and/or material vibrations generated in the impact application device, - an analysis unit which is configured both to calculate a frequency characterization of the vibrations for each impact applied by the impact application device to the prosthetic component, from the corresponding data produced by the at least one vibrational sensor, and to compare the frequency characterizations that are respectively calculated for successive impacts so as to provide at each of the successive impacts either a first indication when the assembly between the prosthetic component and the support member is not fully seated or a second indication when the assembly between the prosthetic component and the support member is fully seated, and - a user interface which provides feedback based on the first and second indications.

US 5,836,891 A describes a non-destructive and non-invasive method and apparatus that determines the structural integrity of discrete pieces of material, such as bone, medical implants and structural parts by determining the impact ratio of a striking mass that impacts and rebounds from the material. The impact ratio is equal to the ratio of instantaneous velocity of the striking mass immediately after the impact to the instantaneous velocity of the striking mass immediately prior to impact. A means can be used to force the striking mass towards the material to impact the material. In this case, the measured history of the displacement during the impact and the rebound are used to compute the impact ratio, the ratio being directly related to the impact reaction of the material. The impact ratio can be used to determine the structural integrity of the material or the onset of osteoporosis. The impact ratio can also be compared with previous measurements of the same piece of material or with standardized values of impact ratios to monitor changes in the impact reaction of the discrete piece of material for the diagnosis of incipient failure in the material and monitor the structural integrity of the piece of material.

SUMMARY OF THE INVENTION

The invention is defined in claim 1 and aspects of the invention are set out in the accompanying dependent claims. Combinations of features from the dependent claims may be combined with features of the independent claims as appropriate .

According to the invention, there is provided a surgical impaction apparatus comprising:
a surgical hammer having:

> a handle;
> an impaction head having an impaction axis and a striking face, wherein the striking face is substantially perpendicular to the impaction axis; and
> an inertial measurement unit operable to measure a direction in which an impaction force is applied by the impaction head relative to the impaction axis.

The surgical hammer may further include a force sensor configured to measure the impaction force applied along the impaction axis.

The inertial measurement unit may be operable to determine to the impaction force. For instance, this may be done by an accelerometer of the inertial measurement unit. The accelerometer may measure a magnitude of vibrations that occur in the surgical hammer when it impacts an object. The peak in these vibrations may be proportional to the magnitude of the impaction force.

The surgical impaction apparatus may include a user feedback part.

The user feedback part may include an array of indicator lights. The array may include a central light operable to indicate when an angle between the direction in which an impaction force is applied by the impaction head and the impaction axis is lower than a threshold value. The array may also include one or more further lights for indicating when the angle between the direction in which the impaction force is applied by the impaction head and the impaction axis is higher than the threshold value.

Each of the plurality of further lights may be distributed at intervals around the central light to indicate a direction in which the impaction force applied by the impaction head deviates from the impaction axis.

The array of lights may be located on a surface of the impaction head opposite the striking face.

The user feedback part may be operable to vibrate the handle to provide tactile feedback in response to a determination that the angle between the direction in which the impaction force is applied by the impaction head and the impaction axis is higher than the threshold value.

The user feedback part may include an audible feedback device. The audible feedback device may be operable to produce an audible indication in response to a determination that the angle between the direction in which the impaction force is applied by the impaction head and the impaction axis is higher than the threshold value.

The user feedback part may include a display. The display may be operable to display at least one indication of the angle between the direction in which the impaction force is applied by the impaction head and the impaction axis. The display may be operable to display at least one indication of a component of the impaction force applied by the impaction head in a direction orthogonal to the impaction axis.

The display may be located on the surgical hammer.

The surgical impaction apparatus may include a processing and display unit separate from the surgical hammer. The display may be included in the processing and display unit.

The inertial measurement unit may be located in the impaction head. The inertial measurement unit may be located in the handle.

The surgical impaction apparatus may include a reference unit separate from the surgical hammer. The reference unit may include second inertial measurement unit. The reference unit may be attachable to an object to be struck by the surgical hammer to act as a reference for measuring the direction in which the impaction force was applied by the impaction head relative to the impaction axis.

A surgical kit can be provided, the surgical kit including a surgical impaction apparatus of the kind described above.

A method is disclosed, of providing feedback to a surgeon about an impaction force applied by an impaction head of a surgical hammer, the method comprising:

> striking an object with a striking face of the impaction head;
> measuring a direction in which an impaction force was applied by the impaction head relative to an impaction axis of the impaction head, wherein the striking face is substantially perpendicular to the impaction axis.

The method may include measuring a component of the impaction force in a direction orthogonal to the impaction axis.

The method may include providing feedback to the surgeon. The feedback may indicate whether an angle between the direction in which the impaction force was applied and the impaction axis was lower than a threshold value. The feedback may indicate a direction in which the impaction force applied by the impaction head deviates from the impaction axis.

The method may include using a second inertial measurement unit attached to the object as a reference for measuring the direction in which the impaction force was applied by the impaction head relative to the impaction axis.

The method may be used for training a surgeon to apply an impaction force in a direction substantially parallel to the impaction axis of the impaction head of the surgical hammer.

BRIEF DESCRIPTION OF THE DRAWINGS

Embodiments of the present invention will be described hereinafter, by way of example only, with reference to the accompanying drawings in which like reference signs relate to like elements and in which:

Figure 1A shows a rear view of a surgical hammer according to an embodiment of this invention;
Figure 1B shows a side view of the surgical hammer of Figure 1A;
Figure 2A shows a rear view of a surgical hammer according to an embodiment of this invention;
Figure 2B shows a side view of the surgical hammer of Figure 2A;
Figure 3A shows a rear view of a surgical hammer according to an embodiment of this invention;
Figure 3B shows a side view of the surgical hammer of Figure 3A;
Figure 4A shows a rear view of a surgical hammer according to an embodiment of this invention;
Figure 4B shows a side view of the surgical hammer of Figure 4A;
Figure 5A shows a rear view of a surgical hammer according to an embodiment of this invention;
Figure 5B shows a side view of the surgical hammer of Figure 5A;
Figure 5C shows a top view of the surgical hammer of Figure 5A;
Figure 6A shows a rear view of a surgical hammer according to an embodiment of this invention;
Figure 6B shows a side view of the surgical hammer of Figure 6A;
Figure 6C shows a portable computing device for use with the surgical hammer of Figure 6A;
Figure 7A shows a rear view of a surgical hammer according to an embodiment of this invention;
Figure 7B shows a side view of the surgical hammer of Figure 7A;
Figure 7C shows a reference unit for use with the surgical hammer of Figure 7A;
Figure 8 schematically shows the components of a surgical hammer according to an embodiment of this invention;
Figure 9 schematically shows the components of a surgical hammer and a processing and display device according to an embodiment of this invention;
Figure 10 schematically shows the components of a surgical hammer, a processing and display device and a reference unit according to an embodiment of this invention;
Figure 11 shows the strike force of a number of strike events according to an embodiment of this invention;

Figure 12 shows the strike magnitude of a number of strike events according to an embodiment of this invention;
Figure 13 plots the peak strike magnitude of a number of strike events against the peak strike force of those events according to an embodiment of this invention;
Figure 14 plots the strike angles (roll, pitch and yaw angles) of a number of notional blows of the surgical hammer;
Figure 15 plots the resolved strike forces of the surgical hammer strikes shown in Figure 14; and
Figure 16 schematically illustrates the orientation of a surgical hammer in a Cartesian reference frame in accordance with an embodiment of this invention.

DETAILED DESCRIPTION

Embodiments of the present invention are described in the following with reference to the accompanying drawings.

As described herein, a surgical impaction apparatus comprises a surgical hammer. The apparatus may also include other components, such as a separate processing and display unit and/or separate reference unit.

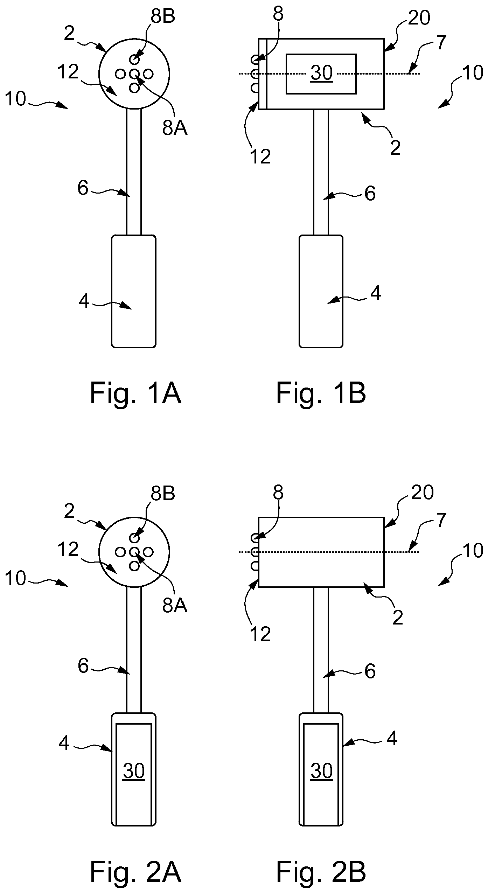

Figure 1A shows a rear view of a surgical hammer 10 according to an embodiment of this invention. Figure 1B shows a side view of the surgical hammer 10 of Figure 1A. The surgical hammer can be used during a surgical procedure for impacting a component. Examples of this include the impaction of a broach, stem head, stem implant or acetabular cup in a hip replacement procedure, the impaction of a tibial tray, tibial trials or trials for a femoral component in a knee replacement procedure, and impaction of components in a shoulder replacement procedure.

The surgical hammer 10 has an impaction head 2. The surgical hammer 10 also has a handle. The handle may include a shaft 6 attached at one end to the impaction head 2. The handle may also include a grip 4 located at an end of the shaft 6 opposite the impaction head 2.

The impaction head 2 has a striking face 20 with which the surgical hammer 10 may be used to strike an object. The striking face 20 may be substantially flat. The striking face 20 is substantially perpendicular to an impaction axis 7 of the impaction head 2. In use, it is intended that when the impaction head 2 strikes an object, it strikes along the impaction axis 7. However, it is envisaged that to apply a force with the surgical hammer 10 in this way may be difficult or require practice or training. In particular, it may not be easy to direct the surgical hammer 10 such that it applies the impaction force directly along the impaction axis 7. This is because, in practice, the striking face 20 of the impaction head 2 may not be exactly square on as it contacts the object. This may in turn lead to misalignments of the object that is being impacted. As will be described in more detail below, embodiments of the invention allow the direction in which an impaction force is applied by the impaction head 2 relative to the impaction axis 7 to be measured. Embodiments of the invention may also provide feedback to the surgeon about the direction of the applied force. This feedback may be used during a surgical procedure by the surgeon to adjust the direction of blows applied by the surgical hammer 10. The feedback may also be used in a training environment to allow the surgeon to practice striking an object such that the force is applied along the impaction axis 7.

The surgical hammer 10 includes an inertial measurement unit (IMU) 30. In this example, the inertial measurement unit 30 is located in the impaction head 2. The inertial measurement unit 30 may comprise one or more sensors such as gyroscopes or accelerometers.

The inertial measurement unit 30 is operable to measure a direction in which an impaction force is applied by the impaction head 2 relative to the impaction axis 7. The direction of the applied force may, for instance, be measured using a gyroscope of the inertial measurement unit 30.

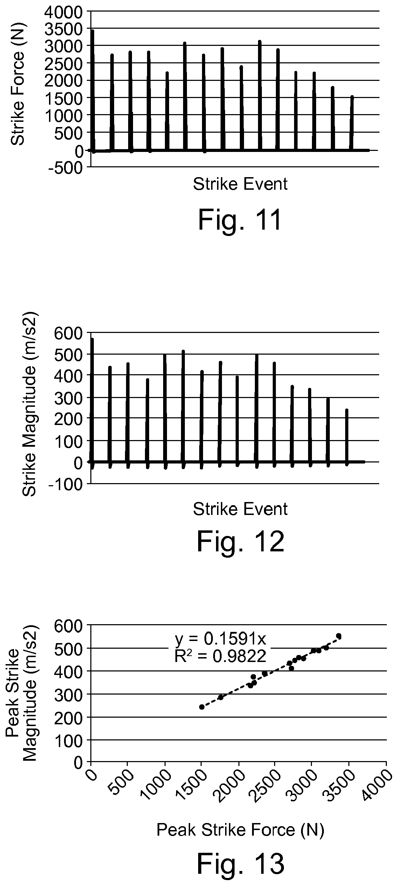

The inertial measurement unit 30 may also be operable to measure the magnitude of the force applied by the surgical hammer 10. In particular, to measure the impaction force magnitude, the inertial measurement unit 30 (e.g. an accelerometer thereof) may measure the magnitude of the vibrations that occur in the surgical hammer 10 when the surgical hammer 10 impacts an object. The peak in these vibrations is proportional to the magnitude of the impaction force applied by the surgical hammer 10, as is demonstrated in Figures 11 to 13, to be described below.

The information may be combined with the direction measurement to provide feedback about both the magnitude and direction of the force applied during a blow of the surgical hammer 10.

The inertial measurement unit 30 may output one or more signals indicative of the direction (and magnitude) of the force applied during a blow of the surgical hammer 10.

As noted above, the surgeon may be provided with feedback regarding the direction (and optional also the magnitude) of the force applied by the surgical hammer 10. The surgical impaction apparatus may include a user feedback part to provide this feedback. In this example, the user feedback part is provided as part of the surgical hammer 10 itself, although, as will be described below, the user feedback part may be provided separately.

The user feedback part in this example comprises an array of indicator lights. The array of indicator lights may be located on a rear surface 12 of the impaction head 2, opposite the striking face 20, although this is not essential. These lights include a central light 8A and one or more further lights 8B. The central light 8A may be located on the impaction axis 7, although this is not essential.

The central light 8A may be operable to turn on to indicate when an angle between the direction in which an impaction force is applied by the impaction head 2 and the impaction axis 7 is lower than a threshold value. The threshold value may, for instance, be fifteen degrees. The threshold value may be adjustable using a user interface of the apparatus.

The one or more further lights 8B may be operable to indicate when an angle between the direction in which an impaction force is applied by the impaction head 2 and the impaction axis 7 is higher than the threshold value. The further lights 8B may be distributed at intervals (e.g. equally spaced) around the central light 8A. In the example of Figure 1, there are four further lights 8B, which are distributed at ninety degree intervals around the central light 8A in the array (it is envisaged that there may be greater or fewer lights 8B, and the that lights 8B may be located at different intervals around the central light 8A). The locations of the further lights 8B may allow them to be selectively turned on to indicate the direction in which the impaction force applied by the impaction head 2 deviated from the impaction axis 7. Where the direction of deviation of the applied force falls between two of the further lights 8B, those two further lights 8B may selectively be switched on to indicate this.

It is envisaged that the lights of the array may be different colours. For example, the central light may be a first colour (e.g. green), for indicating a hammer blow that is applied along, or close to (e.g. at an angle lower than the threshold angle value mentioned above), the impaction axis 7. The one or more further lights 8B may be a second, different colour (e.g. red) for indicating a hammer blow that was applied off axis (e.g. at an angle higher than the threshold angle value mentioned above). It is also envisaged that the lights 8A, 8B may be operable to shine at an intensity that indicates the magnitude of the applied force. It is further envisaged that the lights 8A, 8B associated with a given strike of the surgical hammer 10 may remain illuminated until the next strike of the surgical hammer 10.

It is envisaged that the inertial measurement unit 30 may be located elsewhere in the surgical hammer 10. Figure 2A shows a rear view of a surgical hammer according to another embodiment of this invention. Figure 2B shows a side view of the surgical hammer of Figure 2A. The surgical hammer 10 in this embodiment is similar to the surgical hammer 10 described above in relation to Figures 1A and 1B. However, in this example, the inertial measurement unit 30 is located in the handle (e.g. inside the grip 4.

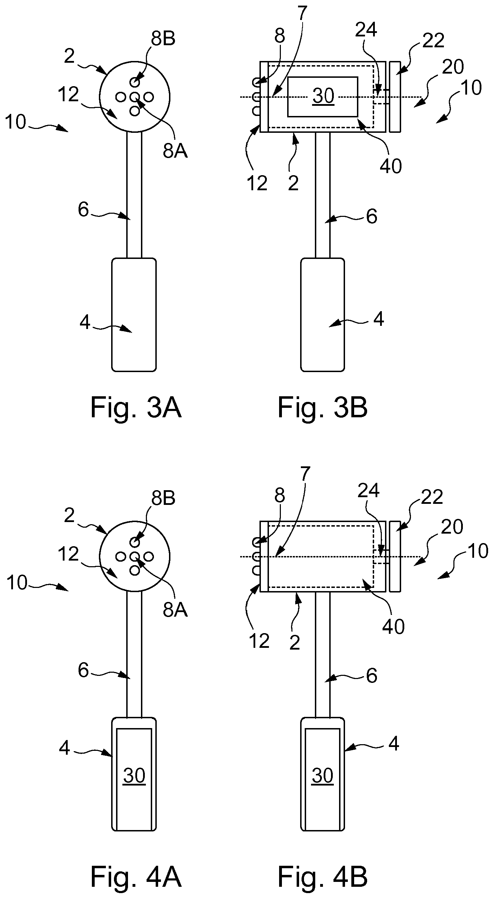

Figure 3A shows a rear view of a surgical hammer 10 according to an embodiment of this invention. Figure 3B shows a side view of the surgical hammer 10 of Figure 3A. The surgical hammer 10 in this example is similar to the surgical hammer 10 described above in relation to Figures 1A and 1B, and only the differences will be described below in detail.

In this embodiment, the surgical hammer 10 includes a force sensor 40. The force sensor 40 may be used to determine the magnitude of the force applied by the impaction head 2, resolved along the impaction axis 7. The output of the force sensor 40 may be combined with the output of the inertial measurement unit 30 to provide information (feedback) to the surgeon about the magnitude and direction of the force applied during a blow of the surgical hammer 10. In the present embodiment, the inertial measurement unit 30 need not provide acceleration information for deriving the magnitude of the applied force, as this may be determined instead from the force sensor 40.

In this embodiment, the force sensor 40 includes an impaction member 22. The striking face 20 of the impaction head 2 may be a surface of the impaction member 22. The impaction member 22 may be located at an end of a shaft 24. The shaft 24 may be located on the impaction axis. The force applied along the shaft 24 may be measured by the force sensor 40, thereby to determine the magnitude of the force applied by the impaction head 2, resolved along the impaction axis 7.

Again, it is envisaged that the inertial measurement unit 30 may be located elsewhere in the surgical hammer 10. Figure 4A shows a rear view of a surgical hammer according to another embodiment of this invention. Figure 4B shows a side view of the surgical hammer of Figure 4A. The surgical hammer 10 in this embodiment is similar to the surgical hammer 10 described above in relation to Figures 3A and 3B. However, in this example, the inertial measurement unit 30 is located in the handle (e.g. inside the grip 4).

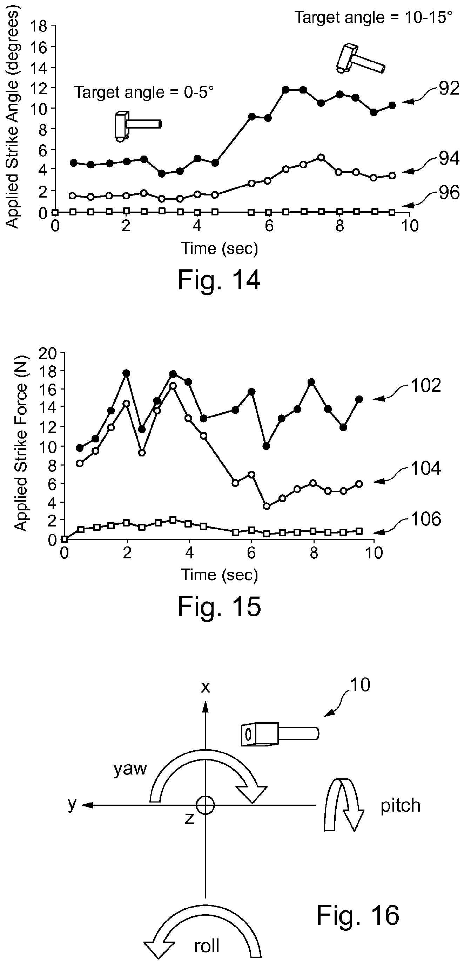

Referring briefly to Figure 16, there is schematically illustrated a surgical hammer 10 oriented within a Cartesian reference frame in accordance with an embodiment of this invention. In this example, it is assumed that the surgical hammer 10 is nominally (i.e. at zero deflection) oriented such that the impaction axis 7 is aligned parallel to the z-axis, with the striking face 20 facing in the negative z-direction and the handle pointing in the negative y-direction. Figure 16 also schematically shows rotations of the surgical hammer 10 within the Cartesian reference frame. In this example, a roll angle of the surgical hammer 10 corresponds to a rotation of the surgical hammer 10 about the x-axis, a pitch angle of the surgical hammer 10 corresponds to a rotation of the surgical hammer 10 about the y-axis, and a yaw angle of the surgical hammer 10 corresponds to a rotation of the surgical hammer 10 about the z-axis.

If the notional direction of the surgical hammer path rotates around the x-axis, the resolved force of interest is that which occurs in the roll orientation (i.e. rotation about the x-axis) as may be given by:

$$\text{Force Vertical} = \text{Force}_{mag} \cdot \text{Sin } \theta_{roll}$$

$$\text{Force Horizontal} = \text{Force}_{mag} \cdot \text{Cos } \theta_{roll}$$

where Force$_{mag}$ is the unresolved force magnitude. If the notional direction of the hammer path rotates around the y-axis, the resolved force of interest is that which occurs in the pitch orientation (i.e. rotation about the y-axis) as may be given by:

$$\text{Force Vertical} = \text{Force}_{mag} \cdot \text{Sin } \theta_{pitch}$$

$$\text{Force Horizontal} = \text{Force}_{mag} \cdot \text{Cos } \theta_{pitch}$$

where Force$_{mag}$ is again the unresolved force magnitude.

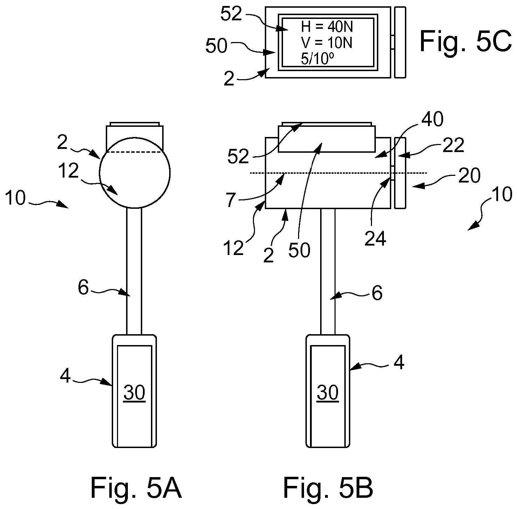

Figure 5A shows a rear view of a surgical hammer according to another embodiment of this invention. Figure 5B shows a side view of the surgical hammer of Figure 5A. Figure 5C shows a top view of the surgical hammer of Figure 5A. The surgical hammer 10 shown in Figure 5 is similar to that shown in Figure 4, and only the differences will be described below in detail.

In this embodiment, the surgical hammer 10 includes a feedback part that is different to the array of lights described above in relation to Figures 1 to 4. In this embodiment, the feedback part comprises a display unit 50 having a display 52 (e.g. a LCD, OLED or other kind of electronic display). The display unit 50 is provided as part of the surgical hammer 10. The display unit 50 may be provided on the impaction head 2 as shown in Figure 5 for ease of reference by the surgeon, although it is envisaged that the display unit 50 may be provided elsewhere (e.g. on the handle). In this embodiment, the display 52 is located on an upper surface of the impaction head 2 opposite the handle. A surface normal of the display 52 is oriented perpendicular the impaction axis 7, but in some examples it may, for instance, be tilted towards the rear surface 12 of the impaction head 2.

As shown in Figure 5C, the display unit 50 may be used to provide feedback to the surgeon about the direction of the force applied by a blow of the surgical hammer 10. This feedback may be alphanumerical.

For instance, the roll angle and pitch angle mentioned above may be shown on the display 52 in the format "$\theta_{pitch}/\theta_{roll}$" (5/10° in example of Figure 5C). As also shown in Figure 5C, the horizontal and vertical force components mentioned above may be shown on the display 52. The display unit 50 may also be used to provide feedback to the surgeon about the unresolved force magnitude (Force$_{mag}$) applied by a blow of the surgical hammer 10.

As with the operation of the array of lights described above, it is further envisaged that the display 52 may continue to show the values associated with a given strike of the surgical hammer 10 until the next strike of the surgical hammer 10.

It is envisaged that the features of the display unit 50 described above may also be used in place of the array of lights in an embodiment of the kind described above in relation to any of Figures 1 to 3.

Instead of providing a display unit 50 on the surgical hammer 10 itself, it is envisaged that the information regarding the direction and/or magnitude of the applied force may be provided on a separate device such as a desktop computer, or portable computing device such as a laptop, tablet, mobile phone or any other kind of computing device. As will be described in more detail below, the surgical hammer and the computing device in such examples may be provided with features for communicating information between them. This information may include data acquired from the inertial measurement unit and/or force sensor, details of the feedback to be shown on a display of the computing device, and control signals for initiating the surgical hammer and/or computing device at start up. The information may be conveyed, for example by a wired connection, or wirelessly (e.g. using a Wi-Fi and/or Bluetooth connection).

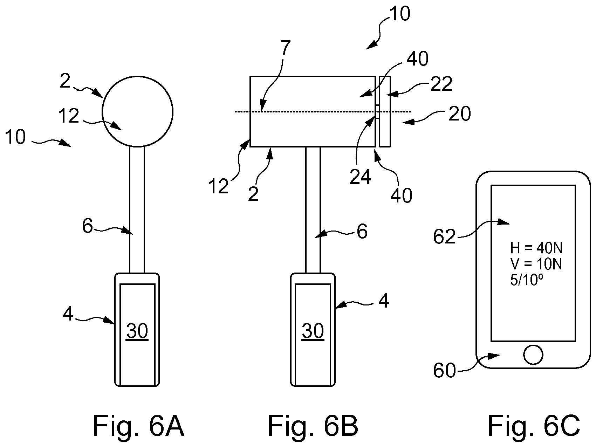

Figure 6A shows a rear view of a surgical hammer 10 according to another embodiment of this invention. Figure 6B shows a side view of the surgical hammer 10 of Figure 6A. Figure 6C shows a portable computing device 60 for use with the surgical hammer 10 of Figure 6A. The surgical hammer 10 in this embodiment is similar to the one shown in Figure 5 and only the differences will be described here in detail.

In this embodiment, instead of providing a display unit 50 on the surgical hammer 10 itself, the information regarding the direction and/or magnitude of the applied force is displayed on a portable computing device 60. The portable computing device 60 includes a display 62. The display 62 may be used to provide feedback to the surgeon in much the same way as described above in relation to the display unit 50 and display 52 of Figure 5.

It is envisaged that the features of the computing device (e.g. portable computing device 60) described above may also be used in place of the array of lights in an embodiment of the kind described above in relation to any of Figures 1 to 4.

In some embodiments, the feedback unit may be operable to provide tactile feedback to the surgeon. For instance, the user feedback part may be operable to vibrate the handle of the surgical hammer 10 in response to a determination that the angle between the direction in which the impaction force is applied by the impaction head 2 and the impaction axis 7 is higher than a threshold value (such as the threshold value described above). In some examples, the user feedback part may provide a different kind of vibration in response to a determination that the angle between the direction in which the impaction force is applied by the impaction head 2 and the impaction axis 7 is lower than the threshold value. The vibrations may be provided by a motor having an eccentric weight attached thereto.

The tactile feedback (e.g. vibrations) may also be provided in apparatus of the kind described above, in addition to the visual feedback provided by the array of lights and/or the displays. In this way a combination of visual and tactile feedback may be provided to the surgeon.

In some embodiments, the user feedback part may comprise an audible feedback device such as a speaker. The audible feedback device may be operable to produce an audible indication in response to a determination that the angle between the direction in which the impaction force is applied by the impaction head 2 and the impaction axis 7 is higher than a threshold value (such as the threshold value described above). In some examples, the user feedback part may provide a different kinds audible feedback (e.g. a buzz having a different tone) in response to a determination that the angle between the direction in which the impaction force is applied by the impaction head 2 and the impaction axis 7 is lower than the threshold value. The audible feedback may include speech. For instance, the audible feedback may verbally specify the direction and/or magnitude of the applied force.

The audible feedback may also be provided in an apparatus of the kind described above, in addition to the visual and/or tactile feedback. In this way a combination of visual, tactile and/or audible feedback may be provided to the surgeon.

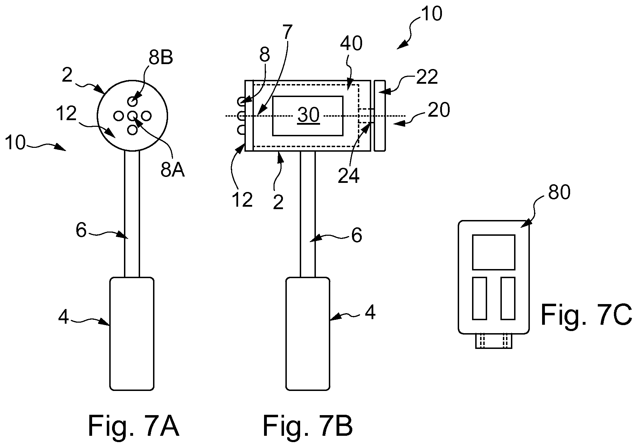

Figure 7A shows a rear view of a surgical hammer 10 according to another embodiment of this invention. Figure 7B shows a side view of the surgical hammer 10 of Figure 7A. Figure 7C shows a separate reference unit 80 for use with the surgical hammer 10 of Figure 7A. The apparatus in this example is similar to that described above in relation to Figure 3B, and only the differences will be described below in detail.

The surgical impaction apparatus in this example includes a reference unit 80. The reference unit 80 includes a second inertial measurement unit. The reference unit 80 may communicate with the surgical hammer 10 and/or with a computing device of the kind described above in relation to Figure 6 using a wired or a wireless (e.g. Wi-Fi, Bluetooth) connection. This may allow the reference unit to be synchronised with the surgical hammer 10 and/or separate computing device, and may allow data collected by the second inertial measurement unit to be communicated to the surgical hammer 10 and/or separate computing device.

The reference unit 80 is attachable to an object (e.g. a facet of a strike surface of) to be struck with the surgical hammer 10. For instance, the reference unit could be attached to a handle of a broach used during a hip replacement procedure, a stem taper during a femoral stem insertion, a cup inserter during cup insertion, and so on. In this embodiment, the reference unit 80 may allow for correction of movements of the strike surface of the object during impaction. In this embodiment, the strike angles can be calculated by determining the relative angle between the inertial measurement unit 30 of the surgical hammer 10 and the inertial measurement unit of the reference unit 80 mounted on the strike surface of the object to be struck with the surgical hammer 10. Geometric calculations can thus be applied to determine the direction of the force applied by the impaction, while taking into account movements of the object to be struck. The angles of the respective inertial measurement units may be measured by gyroscopes of those inertial measurement units.

It is envisaged that a reference unit 80 of the kind described above may be used in conjunction with any of the embodiments described above in relation to Figures 1 to 6.

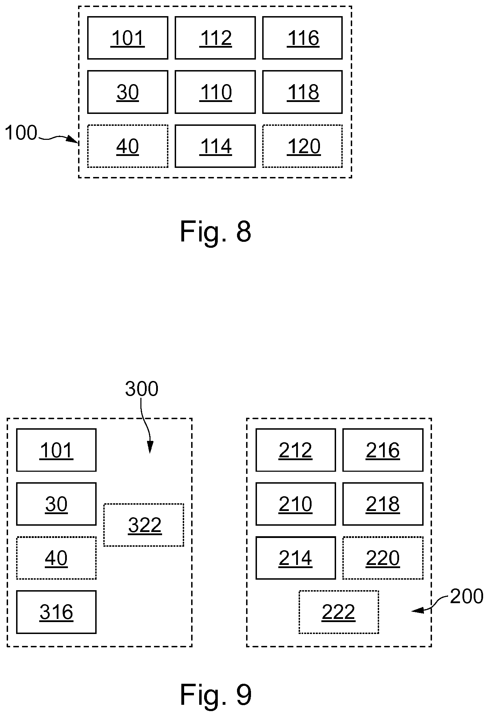

Figure 8 schematically shows the components of a surgical hammer 100 according to an embodiment of this invention. The surgical hammer 100 may, for instance, be a surgical hammer of the kind described above in relation to any of Figures 1 to 5. The components of the surgical hammer 100 such as the handle and the impaction head are represented at 101 in Figure 8. As described above, the surgical hammer 100 includes an inertial measurement unit 30. Optionally, e.g. as described in relation to Figures 3 and 4, the surgical hammer 100 may include a force sensor 40.

The surgical hammer 100 in this example includes a user feedback part 118. This may, for example, be an array of lights, an in-built display unit, a tactile feedback device, and/or an audible feedback device of the kind described above.

The surgical hammer 100 in this example may also include a user interface 120. This may allow for activation, configuration and control of the surgical hammer 100. The user interface 120 may include one or more buttons and/or dials. The user interface may be implemented by the display of the user feedback part. The display may be touch sensitive.

The surgical hammer 100 in this example includes a power supply 116. The power supply may, for example, comprise a battery.

The surgical hammer 100 in this example includes a data acquisition module 112. The data acquisition module 112 may be operable to receive and process data received from outputs of the inertial measurement unit 30 and/or the force sensor 40. The data acquisition module 112 may be responsible for calculating the direction and magnitude of the applied force using data received from the inertial measurement unit 30 and/or the force sensor 40.

The surgical hammer 100 in this example includes a memory 114. The memory 114 may be used to store the data outputted by the inertial measurement unit 30 and or the force sensor 40. The memory 114 may comprise solid state memory. The memory 114 may be removable (e.g. it may comprise a memory card such as an SD memory card).

The surgical hammer 100 in this example includes control logic 110, such a microprocessor or microcontroller. The control logic 110 may be connected to the various features of the surgical hammer 100 to control their operation. For instance, the control logic 110 may be operable to cause the user feedback part to provide feedback according to the direction and magnitude of the force determined by the data acquisition module 112. The control logic 110 may also be operable to adjust the aforementioned threshold value using input provided by the user through the user interface 120.

Figure 9 schematically shows the components of a surgical hammer 300 and a processing and display device 200 according to an embodiment of this invention.

The surgical hammer 300 may, for instance, be a surgical hammer of the kind described above in relation to Figures 6A and 6B. The processing and display device 200 may for instance, be a separate computing device of the kind described above. For instance, the processing and display device 200 may be a portable device 60 of the kind described in relation to Figure 6C.

The components of the surgical hammer such as the handle and the impaction head are represented at 101 in Figure 9. As described above, the surgical hammer 300 includes an inertial measurement unit 30. Optionally, e.g. as described in relation to Figures 3 and 4, the surgical hammer 100 may include a force sensor 40.

The surgical hammer 300 in this example includes a power supply 316. The power supply 316 may, for example, comprise a battery.

The surgical hammer 300 in this example includes connectivity features 322, which may be used to connect the surgical hammer 300 to the processing and display device 200. As noted previously, the connection may be a wired connection, or a wireless connection (e.g. a Wi-Fi and/or Bluetooth connection). The processing and display device 200 may include corresponding connectivity features 222.

The processing and display device 200 in this example includes a power supply 216. The power supply may, for example, comprise a battery.

In this example, some of the components described above in relation to Figure 8 are instead provided in the processing and display device 200. For instance, the processing and display device 200 includes a user feedback part 218, which may, for instance, be a display 62 as described in relation to Figure 6C. The user feedback part 218 may alternatively, or additionally, include an audible feedback device or tactile feedback device of the kind described above.

The processing and display device 200 in this example includes a data acquisition module 212. The data acquisition module 212 may be operable to receive and process data received from outputs of the inertial measurement unit 30 and/or the force sensor 40. The data acquisition module 212 may be responsible for calculating the direction and magnitude of the applied force using data received from the inertial measurement unit 30 and/or the force sensor 40.

The processing and display device 200 in this example includes control logic 210, such a microprocessor or microcontroller. The control logic 210 may be connected to the various features of the processing and display device 200 to control their operation. For instance, the control logic 210 may be operable to cause the user feedback part to provide feedback according to the direction and magnitude of the force determined by the data acquisition module 212.

The processing and display device 200 in this example may include a user interface 220. This may allow for activation, configuration and control of the processing and display device 200 and (remotely) the surgical hammer 300.

The processing and display device 200 in this example may include a memory 214. The memory 214 may be used to store the data outputted by the inertial measurement unit 30 and or the force sensor 40 via the connectivity features 322, 222. The memory 214 may comprise solid state memory. The memory 214 may be removable (e.g. it may comprise a memory card such as an SD memory card).

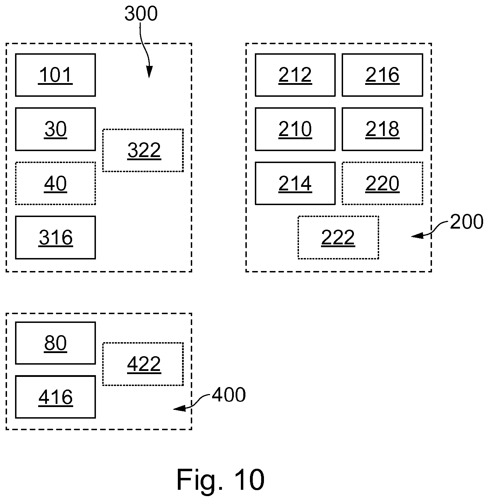

Figure 10 schematically shows the components of a surgical hammer 300, a processing and display device 200 and a reference unit 400 according to an embodiment of this invention. The surgical hammer 300 and processing and display device 200 shown in Figure 10 may be substantially as described above in relation to the surgical hammer 300 and processing and display device 200 shown in Figure 9. The reference unit 400 may, for instance, be a reference unit 400 of the kind described above in relation to Figure 7C.

The reference unit 400 includes a second inertial measurement unit 416, as described above in relation to Figure 7C. The reference unit 400 may also include a power supply 416. The power supply 416 may, for example, comprise a battery.

The reference unit 400 may also include connectivity features 422. As described previously, this may allow the reference unit 400 to communicate with the surgical hammer 300 and/or with the processing and display device 200 using a wired or a wireless (e.g. Wi-Fi, Bluetooth) connection. This may allow the reference unit to be synchronised with the surgical hammer 300 and/or the processing and display device 200, and may allow data collected by the second inertial measurement unit 416 to be communicated to the surgical hammer 10 and/or the processing and display device 200.

Figure 11 shows the strike force (in Newtons) of a number of actual strike events according to an embodiment of this invention, as measured by a force sensor 40 of the kind described above. Figure 12 shows the strike magnitude (in ms$^{-2}$) of those same strike events as measured by an accelerometer of an inertial measurement unit 30 of the kind described above. In particular, as mentioned above, the accelerometer can measure the vibration acceleration associated with the vibrations that occur in the surgical hammer 10 when the surgical hammer 10 impacts an object. The peak in these vibrations is referred to the strike magnitude of each strike, as plotted in Figure 12. Figure 13 plots the peak strike magnitude of the strike events against the peak strike force of those events. As can be seen in Figure 13, there is a clear linear relationship between the peak strike magnitude and the peak strike force (N) of the strike events. This demonstrates that either a force sensor or an accelerometer of an inertial measurement unit may be used to measure the applied force as described previously.

Figure 14 plots the strike angles (roll, pitch and yaw angles) of a notional sequence of simulated blows of the surgical hammer on an immovable object. In the initial strikes of the sequence, the strike angles are relatively low while in the later strikes in the sequence, for illustrative purposes, the strike angles are rather higher. In Figure 14, for each strike of the surgical hammer:

- a roll angle of the hammer (represented by the data points 92) corresponds to a misalignment (rotation) of the hammer about the x axis;
- a pitch angle of the hammer (represented by the data points 94) corresponds to a misalignment (rotation) of the hammer about the y axis; and
- a yaw angle of the hammer (represented by the data points 96) corresponds to a misalignment (rotation) of the hammer about the z axis.

Figure 15 plots the resolved strike forces of the notional sequence of simulated blows of the surgical hammer shown in Figure 14 (in particular the forces associated with the roll angle of the surgical hammer). In Figure 15:

- data points 102 correspond to the unresolved force magnitude (Force$_{mag}$) applied by each strike of the surgical hammer in Figure 14;
- data points 104 correspond to the vertical component (Force$_{mag}$ . Sin $\theta_{roll}$) of the force applied by each strike of the surgical hammer in Figure 14;
- data points 106 correspond to the horizontal component (Force$_{mag}$ . Cos $\theta_{roll}$) of the force applied by each strike of the surgical hammer in Figure 14;

In combination, Figures 14 and 15 demonstrate that the force applied along the direction of interest (typically the impaction axis 7) is reduced when the surgical hammer is misaligned. As described herein, embodiments of this invention can allow a surgeon to be provided with feedback about this reduction is force during training and/or a surgical procedure.

Accordingly, there has been described a surgical impaction apparatus. The surgical impaction apparatus includes a surgical hammer. The surgical hammer has a handle. The surgical hammer also has an impaction head having an impaction axis and a striking face. The striking face is substantially perpendicular to the impaction axis. The surgical hammer further has an inertial measurement unit, which is operable to measure a direction in which an impaction force is applied by the impaction head relative to the impaction axis. A method of providing feedback to a surgeon about an impaction force applied by an impaction head of a surgical hammer. The method includes striking an object with a striking face of the impaction head. The method also includes mmeasuring a direction in which an impaction force was applied by the impaction head relative to an impaction axis of the impaction head. The striking face is substantially perpendicular to the impaction axis.

Although particular embodiments of the invention have been described, it will be appreciated that many modifications/additions and/or substitutions may be made within the scope of the claimed invention.

**Claims**

1. A surgical impaction apparatus comprising:

   a surgical hammer (10, 100, 300) having:

   a handle; and
   an impaction head (2) having an impaction axis (7) and a striking face (20), wherein the striking face is substantially perpendicular to the impaction axis; **characterised in that** the surgical hammer comprises
   an inertial measurement unit (30) configured to measure a direction in

   which an impaction force is applied by the impaction head relative to the impaction axis.

2. The surgical impaction apparatus of claim 1, wherein the surgical hammer further comprises a force sensor (40) configured to measure the impaction force applied along the impaction axis.

3. The surgical impaction apparatus of claim 1 or claim 2, wherein the inertial measurement unit is operable to determine to the impaction force.

4. The surgical impaction apparatus of any preceding claim comprising a user feedback part (118).

5. The surgical impaction apparatus of claim 4, wherein the user feedback part comprises an array of indicator lights having:

   a central light (8A) operable to indicate when an angle between the direction in which an impaction force is applied by the impaction head and the impaction axis is lower than a threshold value; and
   one or more further lights (8B) for indicating when the angle between the direction in which the impaction force is applied by the impaction head and the impaction axis is higher than the threshold value.

6. The surgical impaction apparatus of claim 5, wherein each of the plurality of further lights are distributed at intervals around the central light to indicate a direction in which the impaction force applied by the impaction head deviates from the impaction axis.

7. The surgical impaction apparatus of claim 5 or claim 6, wherein the array of lights is located on a surface (12) of the impaction head opposite the striking face.

8. The surgical impaction apparatus of any of claims 4 to 7, wherein the user feedback part is operable to vibrate the handle to provide tactile feedback in response to a determination that the angle between the direction in which the impaction force is applied by the impaction head and the impaction axis is higher than the threshold value.

9. The surgical impaction apparatus of any of claims 4 to 8, wherein the user feedback part comprises an audible feedback device operable to produce an audible indication in response to a determination that the angle between the direction in which the impaction force is applied by the impaction head and the impaction axis is higher than the threshold value.

10. The surgical impaction apparatus of any of claims 4 to 9, wherein the user feedback part comprises a display (52) operable to display at least one of:

    at least one indication of the angle between the direction in which the impaction force is applied by the impaction head and the impaction axis;
    at least one indication of a component of the impaction force applied by the impaction head in a direction orthogonal to the impaction axis.

11. The surgical impaction apparatus of claim 10, wherein the display is located on the surgical hammer.

12. The surgical impaction apparatus of claim 10, comprising a processing and display unit (60, 200) separate from the surgical hammer, wherein the display (62, 218) is included in the processing and display unit.

13. The surgical impaction apparatus of any preceding claim, wherein the inertial measurement unit is located in the impaction head.

14. The surgical impaction apparatus of any preceding claim, wherein the inertial measurement unit is located in the handle.

**15.** The surgical impaction apparatus of any preceding claim comprising a reference unit (80, 400) separate from the surgical hammer, the reference unit including second inertial measurement unit.

**Patentansprüche**

**1.** Chirurgische Impaktionsvorrichtung, umfassend:

einen chirurgischen Hammer (10, 100, 300) mit:

einem Griff; und
einem Impaktionskopf (2) mit einer Impaktionsachse (7) und einer Schlagfläche (20), wobei die Schlagfläche im Wesentlichen senkrecht zu der Impaktionsachse ist; **dadurch gekennzeichnet, dass** der chirurgische Hammer eine Trägheitsmesseinheit (30) umfasst, die konfiguriert ist, eine Richtung zu messen, in der eine Impaktionskraft durch den Impaktionskopf relativ zu der Impaktionsachse aufgebracht wird.

**2.** Chirurgische Impaktionsvorrichtung nach Anspruch 1, wobei der chirurgische Hammer ferner einen Kraftsensor (40) umfasst, der konfiguriert ist, die Impaktionskraft zu messen, die entlang der Impaktionsachse aufgebracht wird.

**3.** Chirurgische Impaktionsvorrichtung nach Anspruch 1 oder Anspruch 2, wobei die Trägheitsmesseinheit dahingehend operabel ist, die Impaktionskraft zu bestimmen.

**4.** Chirurgische Impaktionsvorrichtung nach einem der vorhergehenden Ansprüche, umfassend einen Benutzer-Feedback-Teil (118).

**5.** Chirurgische Impaktionsvorrichtung nach Anspruch 4, wobei der Benutzer-Feedback-Teil eine Anordnung von Anzeigeleuchten umfasst, die aufweisen:

ein zentrales Licht (8A), das dahingehend operabel ist, anzuzeigen, wenn ein Winkel zwischen der Richtung, in der eine Impraktionskraft durch den Impaktionskopf aufgebracht wird, und der Impaktionsachse kleiner als ein Schwellenwert ist; und
ein oder mehrere weitere Lichter (8B) zum Anzeigen, wenn der Winkel zwischen der Richtung, in der die Impaktionskraft durch den Impaktionskopf aufgebracht wird, und der Impaktionsachse größer als der Schwellenwert ist.

**6.** Chirurgische Impaktionsvorrichtung nach Anspruch 5, wobei jedes der Mehrzahl von weiteren Lichtern in Intervallen um das zentrale Licht herum verteilt sind, um eine Richtung anzuzeigen, in der die Impaktionskraft, die durch den Impaktionskopf aufgebracht wird, von der Impaktionsachse abweicht.

**7.** Chirurgische Impaktionsvorrichtung nach Anspruch 5 oder Anspruch 6, wobei sich die Anordnung von Lichtern auf einer Oberfläche (12) des Impaktionskopfes gegenüberliegend bzw. entgegengesetzt der Schlagfläche befindet.

**8.** Chirurgische Impaktionsvorrichtung nach einem der Ansprüche 4 bis 7, wobei der Benutzer-Feedback-Teil dahingehend operabel ist, den Griff vibrieren zu lassen, um ein taktiles Feedback als Antwort auf eine Bestimmung bereitzustellen, dass der Winkel zwischen der Richtung, in der die Impaktionskraft durch den Impaktionskopf aufgebracht wird, und der Impaktionsachse größer als der Schwellenwert ist.

**9.** Chirurgische Impaktionsvorrichtung nach einem der Ansprüche 4 bis 8, wobei der Benutzer-Feedback-Teil eine Hörbares-Feedback-Vorrichtung umfasst, die dahingehend operabel ist, als Antwort auf eine Bestimmung bereitzustellen, dass der Winkel zwischen der Richtung, in der die Impaktionskraft durch den Impaktionskopf aufgebracht wird, und der Impaktionsachse größer als der Schwellenwert ist.

**10.** Chirurgische Impaktionsvorrichtung nach einem der Ansprüche 4 bis 9, wobei der Benutzer-Feedback-Teil eine Anzeige (52) umfasst, die dahingehend operabel ist, zumindest eines anzuzeigen:

zumindest eine Angabe des Winkels zwischen der Richtung, in der die Impaktionskraft durch den Impaktionskopf aufgebracht wird, und der Impaktionsachse;
zumindest eine Angabe einer Komponente der Impaktionskraft, die durch den Impaktionskopf in einer Richtung orthogonal zu der Impaktionsachse aufgebracht wird.

**11.** Chirurgische Impaktionsvorrichtung nach Anspruch 10, wobei sich die Anzeige an bzw. auf dem chirurgischen Hammer befindet.

**12.** Chirurgische Impaktionsvorrichtung nach Anspruch 10, umfassend eine Verarbeitungs- und Anzeigeeinheit (60, 200), die separat von dem chirurgischen Hammer ist, wobei die Anzeige (62, 218) in der Verarbeitungs- und Anzeigeeinheit enthalten ist.

**13.** Chirurgische Impaktionsvorrichtung nach einem der vorhergehenden Ansprüche, wobei sich die Trägheitsmesseinheit in dem Impaktionskopf befindet.

**14.** Chirurgische Impaktionsvorrichtung nach einem der vorhergehenden Ansprüche, wobei sich die Trägheitsmesseinheit in dem Griff befindet.

15. Chirurgische Impaktionsvorrichtung nach einem der vorhergehenden Ansprüche, umfassend eine Referenzeinheit (80, 400), die separat von dem chirurgischen Hammer ist, wobei die Referenzeinheit eine zweite Trägheitsmesseinheit enthält.

**Revendications**

1. Appareil chirurgical d'impaction comprenant :

   un marteau chirurgical (10, 100, 300) comportant :

   un manche ; et
   une tête d'impaction (2) présentant un axe d'impaction (7) et une face de frappe (20), dans lequel la face de frappe est sensiblement perpendiculaire à l'axe d'impaction ;

   **caractérisé en ce que** le marteau chirurgical comprend une unité de mesure inertielle (30) configurée pour mesurer une direction dans laquelle une force d'impaction est appliquée par la tête d'impaction par rapport à l'axe d'impaction.

2. Appareil chirurgical d'impaction selon la revendication 1, dans lequel le marteau chirurgical comprend en outre un capteur de force (40) configuré pour mesurer la force d'impaction appliquée le long de l'axe d'impaction.

3. Appareil chirurgical d'impaction selon la revendication 1 ou la revendication 2, dans lequel l'unité de mesure inertielle sert à déterminer la force d'impaction.

4. Appareil chirurgical d'impaction selon une quelconque revendication précédente comprenant une partie de rétroaction utilisateur (118).

5. Appareil chirurgical d'impaction selon la revendication 4, dans lequel la partie de rétroaction utilisateur comprend un réseau de lumières d'indication comportant :

   une lumière centrale (8A) servant à indiquer le moment où un angle entre la direction dans laquelle une force d'impaction est appliquée par la tête d'impaction et l'axe d'impaction est inférieur à une valeur seuil ; et
   une ou plusieurs lumières supplémentaires (8B) pour indiquer le moment où l'angle entre la direction dans laquelle la force d'impaction est appliquée par la tête d'impaction et l'axe d'impaction est supérieur à la valeur seuil.

6. Appareil chirurgical d'impaction selon la revendication 5, dans lequel la pluralité de lumières supplémentaires sont chacune réparties à intervalles autour de la lumière centrale pour indiquer une direction dans laquelle la force d'impaction appliquée par la tête d'impaction dévie par rapport à l'axe d'impaction.

7. Appareil chirurgical d'impaction selon la revendication 5 ou revendication 6, dans lequel le réseau de lumières est situé sur une surface (12) de la tête d'impaction opposée à la face de frappe.

8. Appareil chirurgical d'impaction selon l'une quelconque des revendications 4 à 7, dans lequel la partie de rétroaction utilisateur sert à faire vibrer le manche pour fournir une rétroaction tactile en réponse à une détermination que l'angle entre la direction dans laquelle la force d'impaction est appliquée par la tête d'impaction et l'axe d'impaction est supérieur à la valeur seuil.

9. Appareil chirurgical d'impaction selon l'une quelconque des revendications 4 à 8, dans lequel la partie de rétroaction utilisateur comprend un dispositif de rétroaction audible servant à produire une indication audible en réponse à une détermination que l'angle entre la direction dans laquelle la force d'impaction est appliquée par la tête d'impaction et l'axe d'impaction est supérieur à la valeur seuil.

10. Appareil chirurgical d'impaction selon l'une quelconque des revendications 4 à 9, dans lequel la partie de rétroaction utilisateur comprend un dispositif d'affichage (52) servant à afficher au moins l'une parmi :

    au moins une indication de l'angle entre la direction dans laquelle la force d'impaction est appliquée par la tête d'impaction et l'axe d'impaction ;
    au moins une indication d'une composante de la force d'impaction appliquée par la tête d'impaction dans une direction orthogonale à l'axe d'impaction.

11. Appareil chirurgical d'impaction selon la revendication 10, dans lequel le dispositif d'affichage est situé sur le marteau chirurgical.

12. Appareil chirurgical d'impaction selon la revendication 10, comprenant une unité de traitement et d'affichage (60, 200) séparée du marteau chirurgical, dans lequel le dispositif d'affichage (62, 218) est inclus dans l'unité de traitement et d'affichage.

13. Appareil chirurgical d'impaction selon une quelconque revendication précédente, dans lequel l'unité de mesure inertielle est située dans la tête d'impaction.

14. Appareil chirurgical d'impaction selon une quelconque revendication précédente, dans lequel l'unité de mesure inertielle est située dans le manche.

15. Appareil chirurgical d'impaction selon une quelconque revendication précédente comprenant une unité de référence (80, 400) séparée du marteau chirurgical, l'unité de référence incluant une seconde unité de mesure inertielle.

Fig. 1A          Fig. 1B

Fig. 2A          Fig. 2B

Fig. 3A

Fig. 3B

Fig. 4A

Fig. 4B

52

50

2

H = 40N
V = 10N
5/10°

Fig. 5C

2

12

10

6

4

30

Fig. 5A

52

7

12

2

50

40

22

20

10

24

6

4

30

Fig. 5B

10

2

12

10

6

4 30

Fig. 6A

7

10

40

22

12

2

24

40

20

6

4 30

Fig. 6B

62

H = 40N
V = 10N
5/10°

60

Fig. 6C

Fig. 7A

Fig. 7B

Fig. 7C

Fig. 8

Fig. 9

300

| 101 |
| --- |

| 30 |
| --- |

322

40

| 316 |
| --- |

| 212 | | 216 |
| --- | --- | --- |
| 210 | | 218 |
| 214 | | 220 |

222

200

| 80 |
| --- |

422

| 416 |
| --- |

400

Fig. 10

EP 3 740 166 B1

Fig. 11

Fig. 12

Fig. 13

Fig. 14

Fig. 15

Fig. 16

## REFERENCES CITED IN THE DESCRIPTION

### Patent documents cited in the description

- US 20080125671 A
- US 20150282856 A
- EP 2923677 A
- WO 2018085423 A1
- EP 3260088 A1
- US 5836891 A

### Non-patent literature cited in the description

- **MICHEL, A. ; BOSC, R. ; SAILHAN , F. ; VAYRON, R. ; HAIAT, G.** Ex vivo estimation of cementless acetabular cup stability using an impact hammer. *Medical Engineering and Physics,* 2016, vol. 38, 80-86